(19) **Europäisches Patentamt**

**European Patent Office**

**Office européen des brevets**

(11) Veröffentlichungsnummer: **0 226 767 B1**

(12) # EUROPÄISCHE PATENTSCHRIFT

(45) Veröffentlichungstag der Patentschrift: **16.09.92**

(21) Anmeldenummer: **86115243.7**

(22) Anmeldetag: **04.11.86**

(51) Int. Cl.5: **G01N 33/52**, G01N 21/77, G01N 21/59, G01N 31/22

(54) **Transparentes Teststreifensystem.**

(30) Priorität: **15.11.85 DE 3540526**

(43) Veröffentlichungstag der Anmeldung:
**01.07.87 Patentblatt 87/27**

(45) Bekanntmachung des Hinweises auf die
Patenterteilung:
**16.09.92 Patentblatt 92/38**

(84) Benannte Vertragsstaaten:
**AT BE CH DE FR GB IT LI NL SE**

(56) Entgegenhaltungen:
**EP-A- 0 064 710**    **EP-A- 0 134 953**
**CH-A- 639 491**    **DE-A- 2 502 013**
**DE-A- 2 854 342**    **DE-A- 2 910 134**
**DE-A- 3 215 983**    **DE-A- 3 235 658**

(73) Patentinhaber: **BAYER AG**

**W-5090 Leverkusen 1 Bayerwerk(DE)**

(72) Erfinder: **Hildenbrand, Karlheinz, Dr.**
**Gatzenstr. 147**
**W-4150 Krefeld 1(DE)**
Erfinder: **von Döhren, Hans-Hagen, Dr.**
**Im Mühlenkamp 1**
**W-4630 Bochum-Langendreer(DE)**
Erfinder: **Perrey, Hermann, Dr.**
**Auf der Rheinaue 8**
**W-4150 Krefeld 11(DE)**
Erfinder: **Wehling, Klaus, Dr.**
**Am Rohm 167**
**W-5600 Wuppertal 1(DE)**

## Beschreibung

Die vorliegende Erfindung betrifft ein transparentes Teststreifensystem mit einem ionischen Polyurethan als Filmbildner zur transmissionsphotometrischen Bestimmung einer Komponente einer Flüssigkeit, insbesondere einer Körperflüssigkeit.

Die Bestimmung einer Komponente in einer flüssigen Probe mit Hilfe von Teststreifen gehört in der diagnostischen Chemie zu den etablierten Verfahren. Derartige Analysen sind im Vergleich zu den konventionellen naßchemischen Methoden schneller und einfacher in der Durchführung. Dagegen werden die in der Naßchemie üblichen Genauigkeiten mit Teststreifen in der Regel noch nicht erreicht. Es wurden daher viele Versuche unternommen, diese Nachteile zu beheben (EP 0 064 710, DOS 3 130 749, DOS 2 332 760, DOS 2 532 918, DOS 2 602 975, DOS 3 016 618). Im Rahmen dieser Entwicklungen spielen insbesondere die Struktur der Trägermatrix sowie die Auswertmethode eine wichtige Rolle. Gute Fortschritte brachte hierbei der Ersatz von Papier durch synthetische Polymere als Trägermaterial. Solche Systeme eignen sich bei geeignetem Strukturaufbau auch direkt für die Applikation von Vollblut. Bei den bisher bekannten polymeren Teststreifensystemen für die Vollblutanalyse erfolgt die Abtrennung der roten Blutzellen über mikroporöse Strukturelemente. Das Serum kann dagegen in den Reaktionsraum der Matrix eindringen, wo es zu einer spezifischen Farbreaktion kommt, die beispielsweise nach Abwischen der roten Blutzellen ausgewertet werden kann.

Der strukturelle Aufbau derartiger Testelemente ist jedoch in der Regel relativ kompliziert, was auch die reproduzierbare Herstellung erschwert.

Für die Auswertung der Teststreifen-Reaktionen haben sich neben visuellem Vergleich insbesondere remissionsphotometrische Bestimmungsverfahren durchgesetzt. Derartige reflektometrische Bestimmungen beruhen jedoch auf physikalischen Näherungsmethoden (Kubelka-Munk-Theorie) und bieten nicht die Präzision der in der Naßchemie verwendeten Transmissionsbestimmung (Lambert-Beersches-Gesetz).

Die vorliegende Erfindung beschreibt ein transparentes diagnostisches Mittel, das sich durch einen einfachen Strukturaufbau und eine einfache Herstellbarkeit auszeichnet sowie eine transmissionsphotometrische Auswertung erlaubt.

Die Erfindung betrifft transparente Reagenzträgerschichten in analytischen Mitteln zum trockenchemischen Nachweis von Bestandteilen wäßriger Probelösungen, wobei die Reagenzträgerschicht aus einer in Wasser löslichen oder in Wasser quellbaren Komponente und einer im wesentlichen wasserunlöslichen, filmbildenden ionischen Polyurethan besteht.

Es war bekannt, daß aus wäßrigen Dispersionen in Gegenwart unlöslicher Filmöffner (DOS 29 10 134) wasserfeste, saugende Teststreifen-Matrizes hergestellt werden können.

Durch die eingeschlossenen Füllstoffpartikel werden jedoch opake, poröse Filme erhalten, die für Transmissions-Auswertungen nicht geeignet sind.

Es wurde nun völlig überraschend gefunden, daß transparente, flüssigkeitsaufnehmende Filmsysteme erhalten werden, wenn man zur Herstellung der Filme Gießlösungen verwendet, die aus einem Gemisch einer Dispersion oder Lösung eines filmbildenden ionischen Polyurethans, welches im wesentlichen wasserunlöslich ist, und einer wasserquellbaren bzw. wasserlöslichen Komponente bestehen.

Trägt man derartige Gießlösungen auf gut haftende Unterlagen auf und trocknet, so erhält man porenfreie Filme, die aufgrund ihres Quellvermögens definierte Flüssigkeitsmengen aufnehmen. Das Quellverhalten der Filmsysteme läßt sich durch das Verhältnis von filmbildendem Polymer zu wasserquellbarer Komponente variieren. Bringt man die für eine bestimmte Nachweisreaktion erforderlichen Reagenzien in die Gießlösung ein, so werden nach Beschichten einer Unterlage und Trocknen die erfindungsgemäßen, transparenten Nachweiselemente erhalten.

Das Einarbeiten der Nachweisreagenzien kann je nach Löslichkeit der Reagenzien und Lösungsmittel des polymeren Matrixsystems auf unterschiedliche Art erfolgen. Sind die Nachweisreagenzien im Lösungsmittelsystem der Matrix-Gießlösung löslich, so kann dementsprechend in homogener Phase gearbeitet werden. So werden beispielsweise wasserlösliche Nachweisreagenzien in wässrige Gießlösungen dadurch eingearbeitet, daß die Reagenzien in Wasser gelöst und dann ohne weitere Hilfsmittel in die Matrix-Gießlösung eingerührt werden.

Sind die Nachweisreagenzien im Lösungsmittelsystem der Gießlösung nicht löslich, so können die an sich bekannten Emulsionstechniken (siehe z. B. "Emulsionen", Ullmann, Enzyklopädie der technischen Chemie, 10, 449-473, Verlag Chemie (1975) angewandt werden. Die beiden wichtigsten Emulsionstechniken sind die mechanische und die chemische Emulgierung. Die mechanische Emulgierung kann beispielsweise mit Hilfe schnellaufende Rührwerke durchgeführt werden, wobei häufig noch Hilfslösungsmittel und Emulgatoren zugesetzt werden. Bei der chemischen Emulgierung werden ein oder mehrere Emulgatoren eingesetzt, wobei je nach den entsprechenden HLB-Werten (Hydrophilic-Lipophilic-Balance) Öl in Wasser (Ö/W) der Wasser in Öl (W/O)-Emulsionen erhalten werden können. Geeignete Emulgatoren sind ebenfalls in der obengenannten Literaturstelle aufgeführt. In diesem Zusammenhang sei erwähnt, daß die für die erfindungsgemäßen Nachweiselemente bevorzugt eingesetzten ionischen Polyurethan-Dispersionen selbst Emulgatoreigenschaften besitzen.

Als filmbildende Komponente der Gießlösung sind geeignet Polyurethandispersionen, die ionisch sind, wobei die ionischen Reste Sulfonat-, Carboxylat- oder Ammonium-Gruppen sein können.

Als zweite Polymerkomponente ("Quellkomponente") kommen die bekannten wasserlöslichen bzw. wasserquellbaren Polymeren in Frage, wie zum Beispiel Polyacrylamide, Polyacrylsäuren, Celluloseether, Polyethylenimin, Polyvinylalkohol, Copolymere aus Vinylalkohol und Vinylacetat, Gelatine, Agarose, Alginate sowie Polyvinylpyrrolidon, das sich besonders bewährt hat.

Zum Herstellen der Gießlosung für die erfindungsgemäßen transparenten Teststreifen werden die Lösung bzw. Dispersion der filmbildenden Komponente mit der Lösung bzw. Dispersion der Quellkomponente gemischt, diese Mischung auf einen Träger aufgebracht und getrocknet. Typisch für die vorliegende Erfindung ist, daß das Löse- bzw. Verdünnungsmittel der filmbildenden Komponente und das der Quellkomponente miteinander mischbar, in der Regel sogar identisch sind.

So kann man geeignete Gießlösungen aus organischen Lösemitteln dadurch erhalten, daß man z. B. eine Lösung von Celluloseacetat in Methylenchlorid/Methanol (Filmbildner) mit einer Lösung von Celluloseether (lösliche bzw. quellbare Komponente) in Methylenchlorid/Methanol miteinander mischt.

Bei den bevorzugten wäßrigen Gießlösungen werden wäßrige Polymerdispersionen mit der wäßrigen Lösung der Quellkomponente gemischt, wie z.B. Polyvinylacetat-Dispersionen mit Celluloseethern, Polyurethandispersionen mit Polyvinylalkohol, Polyurethandispersionen mit Gelatine oder Polyurethandispersionen mit Polyvinylpyrrolidon, die sich ganz besonders bewährt haben. Den Gießlösungen können auch Vernetzungsmittel zugesetzt werden, die zu einer erhöhten Wasserfestigkeit der Reagenzträgerschichten führen.

Das Verhältnis von filmbildender Komponente zu quellender Komponente ist ein wichtiger Parameter für die erforderliche Funktionsweise der erfindungsgemäßen Testsysteme. So konnten aus Gießlösungen, bei denen die Quellkomponente fehlte, keine geeigneten Nachweiselemente erhalten werden. Es wurde keine oder nur eine äußerst langsam verlaufende Reaktion beobachtet. Dagegen führen zu hohe Anteile der Quellkomponente nach Probenaufgabe zu starken Trübungen bzw. zu Ablöseerscheinungen beim Abwischen des Probenüberschusses. Das Gewichtsverhältnis von filmbildender zu quellender Komponente hängt von den verwendeten Polymeren sowie vom vorgesehenen Verwendungszweck ab. Es kann 50:1 bis 1:1, bevorzugt 10:1 bis 5:1 betragen, wobei die filmbildende Komponente im Überschuß eingesetzt wird.

Als Trägermaterial für die erfindungsgemäßen transparenten Reagenzträgerschichten sind transparente Kunststoff-Folien, wie zum Beispiel aus Polyethylenteraphthalat geeignet. Bei Verwendung reflektierender Trägerfolien können die Nachweiselemente auch reflektometrisch ausgewertet werden.

Die erfindungsgemäßen Mittel werden üblicherweise als Einschichtsysteme eingesetzt. Sie können jedoch auch als Teil eines Mehrschichtensystems verwendet werden. In den nachfolgenden Beispielen werden die erfindungsgemäßen diagnostischen Nachweismittel näher beschrieben.

Beispiel 1

Zum Nachweis von Glucose in Harn oder Vollblut wurde eine Gieß-Lösung folgender Zusammensetzung hergestellt:

| | |
|---|---|
| 28,9 g | anionische Polyurethandispersion (40 % in Wasser) |
| 0,3 g | 3,3',5,5'-Tetramethylbenzidin gelöst in 1 ml Dimethylformamid |
| 10,6 g | Polyvinylpyrrolidon (MG 350.000), 20 % Lösung in Phosphatpuffer pH 5,5 |
| 0,2 g | 5 % Ascorbinsäure in Wasser |
| 18 KU | Glucoseoxidase ) |
| 72 KU | Peroxidase ) in 10 ml Phosphatpuffer pH 5,5 |
| 0,2 g | Natriumdioctylsulfosuccinat |

Bei der anionischen Polyurethandispersion handelt es sich um ein Umsetzungsprodukt aus

| | |
|---|---|
| 82 Teilen | eines Polyesters aus Adipinsäure, Hexandiol und Neopentylglykol (MG = 1700) |
| 15 Teilen | Hexamethylendiisocyanat |
| 2 Teilen | Na-Ethylendiamin-ethylsulfonat und |
| 1 Teil | Ethylendiamin. |

Das Einarbeiten des 3,3',5,5'-Tetramethylbenzidins erfolgte mit Hilfe eines schnelldrehenden Rührers in die anionische Polyurethandispersion, wobei Dimethylformamid als Hilfslösungsmittel diente.

Die Gießlösung wurde in einer Schichtdicke von 150 $\mu$m auf eine Polyethylenterephthalat-Folie beschichtet und mit Warmuluft getrocknet. Dabei wurde ein auf der Folie haftendes transparentes Teststreifensystem erhalten.

Test mit wäßrigen Glucose-Lösungen (0,1; 0,5; 1,0 %):

Die Glucose-Lösungen wurden auf die Teststreifenoberfläche gegeben und nach 15 sec mit Watte abgewischt. Es entstanden innerhalt 30 sec transparente Blaufärbungen, die entsprechend der steigenden Glucose-Konzentration zunehmend intensiver waren und eine deutliche Differenzierbarkeit ermöglichten.

Test mit Vollblut:

Ein Bluttropfen wurde auf die Teststreifenoberfläche gegeben und nach einer Verweilzeit von 15 sec mit Watte abgewischt. Es war eine homogene, transparente Blaufärbung entstanden.

Transmissionsphotometrische Auswertung:

Testflüssigkeiten:
Wäßrige Glucose-Lösungen mit unterschiedlicher Glucosekonzentration (0, 50, 100, 200, 300, 500, 800, 1000 mg/dl).
Entsprechend der zunehmenden Glucose-Konzentrationen wurden steigende Extinktionswerte gemessen, die im Bereich 0 bis 800 mg/dl eine lineare Abhängigkeit zeigten. Die Wellenlänge der Meßstrahlung betrug 640 nm.
In den folgenden Beispielen sind weitere Gießlösungen beschrieben, aus denen geeignete transparente Glucose-Teststreifen hergestellt wurden. Die Reagenzieneinarbeitung, die Filmherstellung und die Prüfung erfolgte wie in Beispiel 1.

Beispiel 2

Filmbildende Komponente:
25 g   anionische Polyurethandispersion (s. Bsp. 1)
90 g   Hydroxyethyl-Cellulose-Lösung (2 proz. in Wasser, Tylose H 300, Fa. Hoechst AG).

Beispiel 3

Zweischichtiges, transparentes Glucose-Nachweissystem
a) Gießlösung für die Chromogenschicht:
10 g anionische Polyurethandispersion (s. Bsp. 1)
0.08 g 3,3',5,5'-Tetramethylbenzidin
0.07 ml Ascorbinsäure (20 proz. in $H_2O$)
0.43 g Polyvinylpyrrolidon-Lösung (20 proz. in Phosphatpuffer pH 5.5)
0.04 g Na-dioctylsulfosuccinat
b) Gießlösung für die Enzymschicht:
10 g anionische Polyurethandispersion (s. Bsp. 1)
0.43 g Polyvinylpyrrolidon-Lösung (20 proz. in Phosphatpuffer pH 5.5)

0.35 ml Glucoseoxidase-Lösung (1374 U/ml)
8.0 mg Peroxidase (60 U/mg)
0.04 g Na-dioctylsulfosuccinat
Die Gießlösungen a) und b) wurden nacheinander auf Polyethylenterephthalat-Folie beschichtet und getrocknet. Beim Test mit wäßrigen Glucoselösungen wurden in Analogie zu Beispiel 1 konzentrationsabhängige Blaufärbungen beobachtet.

## Patentansprüche

1.   Transparente Reagenzträgerschichten in analytischen Mitteln zum trockenchemischen Nachweis von Bestandteilen wäßriger Probelösungen mit einer Reagenzträgerschicht aus einer in Wasser löslichen oder in Wasser quellbaren Komponente und einer im wesentlichen wasserunlöslichen, filmbildenden Komponente besteht, dadurch gekennzeichnet, daß die filmbildende Komponente ein ionisches Polyurethan ist.

2.   Reagenzträgerschicht nach Anspruch 1, dadurch gekennzeichnet daß die filmbildende Komponente ein ionisches Polyurethan ist, welches als ionische Reste Sulfonat-, Carboxylat- und/oder Ammonium-Gruppen trägt.

3.   Reagenzträgerschicht nach den Ansprüchen 1 und 2, dadurch gekennzeichnet, daß die in Wasser lösliche oder in Wasser quellbare Komponente ein Polyacrylamid, eine Polyacrylsäure, Celluloseether, Polyethylenimin, Polyvinylalkohol, ein Copolymer aus Vinylalkohol und Vinylacetat, Gelatine, Agarose, Polyvinylpyrrolidon oder Alginat sein kann.

4.   Reagenzträgerschicht nach den Ansprüchen 1 bis 3, dadurch gekennzeichnet, daß das Gewichtsverhältnis von filmbildender zur wasserlöslichen oder wasserquellbaren Komponente 50:1 bis 1:1 beträgt.

5.   Reagenzträgerschicht nach Anspruch 4, dadurch gekennzeichnet, daß das Gewichtsverhältnis 10:1 bis 5:1 beträgt.

6.   Verfahren zur Herstellung einer Reagenzträgerschicht nach den Ansprüchen 1 bis 5, dadurch gekennzeichnet, daß man eine Lösung oder Dispersion einer oder mehrerer filmbildender Komponenten mit einer Lösung oder Dispersion einer oder mehrerer in Wasser löslicher oder in Wasser quellbarer Komponenten miteinander mischt und diese Mischung auf einen Träger aufbringt und trocknet.

7.   Verfahren nach Anspruch 6, dadurch gekennzeichnet, daß die Lösemittel oder Verdünnungsmittel, die zur Herstellung der Lösungen oder Dispersionen der filmbildenden Komponente und der in Wasser löslichen oder in Wasser quellbaren Komponente eingesetzt werden, untereinander mischbar sind.

8. Verfahren nach den Ansprüchen 6 und 7, dadurch gekennzeichnet, daß zur Herstellung der Lösung oder Dispersion der filmbildenden Komponente das gleiche Lösemittel oder Verdünnungsmittel verwendet wird, wie zur Herstellung der Lösung oder Dispersion der in Wasser löslichen oder in Wasser quellbaren Komponente.

9. Analytisches Mittel zum trockenchemischen Nachweis von Bestandteilen wäßriger Prolelösungen, dadurch gekennzeichnet, daß es aus wenigstens einer Reagenzträgerschicht nach Ansprüchen 1 bis 5 und eines oder mehrerer Nachweisreagenzien für die Bestimmung des nachzuweisenden Bestandteils besteht.

10. Verfahren zum trockenchemischen Nachweis von Bestandteilen einer wäßrigen Probelösung, dadurch gekennzeichnet, daß man die Probe mit einem analytischen Mittel gemäß Anspruch 9 in Kontakt bringt und die auftretende Nachweisreaktion verfolgt.

11. Verfahren nach Anspruch 10, dadurch gekennzeichnet, daß die Nachweisreaktion transmissionsphotometrisch verfolgt wird.

**Claims**

1. Transparent reagent carrier layers in analytical agents for dry chemical detection of constituents of aqueous sample solutions, the reagent carrier layer consisting of a water-soluble or water-swellable component and an essentially water-insoluble film-forming component, characterised in that the film-forming component is an ionic polyurethane.

2. Reagent carrier layer according to Claim 1, characterised in that the film-forming component is an ionic polyurethane which carries sulphonate, carboxylate and/or ammonium groups as ionic radicals.

3. Reagent carrier layer according to Claims 1 and 2, characterised in that the water-soluble or water-swellable component can be a polyacrylamide, a polyacrylic acid, cellulose ether, polyethyleneimine, polyvinyl alcohol, a copolymer of vinyl alcohol and vinyl acetate, gelatine, agarose, polyvinylpyrrolidone or alginate.

4. Reagent carrier layer according to Claims 1 to 3, characterised in that the weight ratio of film-forming to water-soluble or water-swellable component is 50:1 to 1:1.

5. Reagent carrier layer according to Claim 4, characterised in that the weight ratio is 10:1 to 5:1.

6. Process for the preparation of a reagent carrier layer according to Claims 1 to 5, characterised in that a solution or dispersion of one or more film-forming components and a solution or dispersion of one or more water-soluble or water-swellable components are mixed with one another and this mixture is applied to a carrier and dried.

7. Process according to Claim 6, characterised in that the solvents or diluents used for the preparation of the solutions or dispersions of the film-forming component and the water-soluble or water-swellable component are miscible with one another.

8. Process according to Claims 6 and 7, characterised in that the same solvent or diluent as for the preparation of the solution or dispersion of the water-soluble or water-swellable component is used to prepare the solution or dispersion of the film-forming component.

9. Analytical agent for dry chemical detection of constituents of aqueous sample solutions, characterised in that it consists of at least one reagent carrier layer according to Claims 1 to 5 and one or more detection reagents for determination of the constituent to be detected.

10. Method of dry chemical detection of constituents of an aqueous sample solution, characterised in that the sample is brought into contact with an analytical agent according to Claim 9 and the detection reaction which occurs is monitored.

11. Method according to Claim 10, characterised in that the detection reaction is monitored by transmission photometry.

**Revendications**

1. Couches transparentes de support de réactifs dans des milieux analytiques pour la détection chimique à sec de constituants de solutions aqueuses à analyser, comprenant une couche de support de réactifs formée d'un composant soluble dans l'eau ou gonflant dans l'eau et d'un composant filmogène essentiellement insoluble dans l'eau, caractérisées en ce que le composant filmogène est un polyuréthanne ionique.

2. Couche de support de réactifs suivant la revendication 1, caractérisée en ce que le composant filmogène est un polyuréthanne ionique qui porte comme restes ioniques des groupes sulfonate, carboxylate et/ou ammonium.

3. Couche de support de réactifs suivant les revendications 1 et 2, caractérisée en ce que le composant soluble dans l'eau ou gonflant dans l'eau est un polyacrylamide, un polymère d'acide acrylique, un éther de cellulose, une polyéthylène-imine, un polymère d'alcool vinylique, un copolymère d'alcool vinylique et d'acétate de vinyle, la gélatine, l'agarose, la polyvinylpyrrolidone ou un alginate.

4. Couche de support de réactifs suivant les revendications 1 à 3, caractérisée en ce que le rapport en poids du composant filmogène au composant soluble ou gonflant dans l'eau a une valeur de 50:1 à 1:1.

5. Couche de support de réactifs suivant la revendication 4, caractérisée en ce que le rapport en poids va de 10:1 à 5:1.

6. Procédé de production d'une couche de support de réactifs suivant les revendications 1 à 5, caractérisé en ce qu'on mélange ensemble une solution ou dispersion d'un ou plusieurs composants filmogènes avec une solution ou dispersion d'un ou plusieurs composants solubles dans l'eau ou gonflant dans l'eau et on applique ce mélange sur un support et on le sèche.

7. Procédé suivant la revendication 6, caractérisé en ce que les solvants ou diluants qui sont utilisés pour la production des solutions ou dispersions du composant filmogène et du composant soluble dans l'eau ou gonflant dans l'eau sont miscibles entre eux.

8. Procédé suivant les revendications 6 et 7, caractérisé en ce qu'on utilise, pour préparer la solution ou dispersion du composant filmogène, le même solvant ou diluant que celui que l'on utilise pour préparer la solution ou dispersion du composant soluble dans l'eau ou gonflant dans l'eau.

9. Milieu analytique pour la détection chimique à sec de constituants de solutions aqueuses à analyser, caractérisé en ce qu'il est constitué d'au moins une couche de support de réactifs suivant les revendications 1 à 5 et d'un ou plusieurs réactifs de détection pour le dosage du constituant à détecter.

10. Procédé de détection chimique à sec de constituants d'une solution aqueuse à analyser, caractérisé en ce qu'on met l'échantillon en contact avec un milieu analytique suivant la revendication 9 et on suit la réaction de détection qui se produit.

11. Procédé suivant la revendication 10, caractérisé en ce qu'on suit la réaction de détection par photométrie de transmission.